(19) Europäisches Patentamt
European Patent Office
Office européen des brevets

(11) Publication number: **0 021 606**
**B1**

(12) **EUROPEAN PATENT SPECIFICATION**

(45) Date of publication of patent specification: **09.05.84**

(51) Int. Cl.[3]: **B 01 J 19/18** //B04B5/00, C12N11/00

(21) Application number: **80301725.0**

(22) Date of filing: **23.05.80**

(54) **Apparatus and process for treating a liquid material while it is subjected to a centrifugal force.**

(30) Priority: **01.06.79 GB 7919251**

(43) Date of publication of application:
**07.01.81 Bulletin 81/1**

(45) Publication of the grant of the patent:
**09.05.84 Bulletin 84/19**

(84) Designated Contracting States:
**AT BE CH DE FR GB IT LI NL SE**

(56) References cited:
**AT - B - 296 905**
**DE - A - 2 017 351**
**DE - A - 2 336 591**
**FR - A - 1 202 388**

(73) Proprietor: **IMPERIAL CHEMICAL INDUSTRIES PLC**
**Imperial Chemical House Millbank**
**London SW1P 3JF (GB)**

(72) Inventor: **Steel, Margaret Lilian**
**21 Bunbury Drive**
**Runcorn, Cheshire (GB)**
Inventor: **Norton-Berry, Philip**
**Tan-y-Coed**
**Llandegla Clwyd, Wales (GB)**

(74) Representative: **Bate, Bernard James et al,**
**Imperial Chemical Industries PLC Legal Department: Patents PO Box 6**
**Welwyn Garden City Herts, AL7 1HD (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 021 606 B1

## Description

This invention relates to apparatus for treating a liquid material, particularly to apparatus for treating a liquid material while the liquid material is subjected to a centrifugal force, and to the use of such apparatus.

DE—A—2 336 591 describes a centrifugal reactor for reducing spent ferric chloride electrolyte solution, using iron particles. The reactor comprises an open-top basket reaction chamber with a cylindrical perforated side wall having a plurality of spaced openings which are smaller in size than the size of the iron particles. As the electrolyte and iron particles are fed together to the rotating basket, the solids are filtered out and held against the perforated wall until they are consumed by the flowing electrolyte, replenishing iron particles being fed to the basket at the rate they are consumed.

EP—A—0 002 568 discloses apparatus for effecting mass transfer between two fluid phases by impelling them through a permeable element whilst subjecting them to centrifugal force. We have now found that in such centrifugal apparatus, there may usefully be provided a permeable element which interacts with a liquid material, or portion thereof, while the latter is impelled therethrough, and that by selection of suitable permeable elements, liquids, suspensions, dispersions, and slurries may all be treatable in such apparatus.

Accordingly, a first aspect of the invention provides an apparatus for treating a liquid material, which comprises a permeable element of annular section which is rotatable about the axis of the annulus, means to charge the liquid material to the inner surface of the permeable element and means to collect the liquid material or a component or derivative thereof discharged from the outer surface of the permeable element by rotation of the element, characterised in that the permeable element (a) comprises a catalyst for carrrying out a chemical reaction in or on the liquid material or is formed from a chromatographic support material, and (b) allows the liquid material to flow through its pores.

A further aspect of the invention provides a process for treating a liquid material which process comprises (i) charging the liquid material to a permeable element which allows passage of the liquid material through the pores thereof, the wall surfaces of the pores providing a substantially continuous path over which the liquid material may flow, (ii) rotating the permeable element about an axis to subject the liquid material to a mean acceleration of at least 150 m $sec^{-2}$ as it flows through the said pores away from the said axis and (iii) collecting the liquid material or a component or derivative thereof from the permeable element, characterised in that the permeable element (a) comprises a catalyst for carrying out a chemical reaction in or on the liquid material or is formed from a chromatographic support material, and (b) allows the liquid material to flow through its pores.

The liquid material to be treated in apparatus according to the present invention may, for example, be a neat liquid, a mixture of miscible or immiscible liquids, a solution of one or more solids or gases in a liquid, a foam or a dispersion, suspension or slurry of one or more solids in a liquid. Within the term solid we include biological material, e.g. living and dead cells, which cells may include those derived from plants, animals, bacteria or fungi.

By "interact" we mean that the permeable element catalyses a reaction in or on the liquid material or a portion thereof or it physically or chemically bonds to the liquid material or a portion thereof.

By "permeable element" we mean an element of an apparatus according to the invention which (a) allows passage of the liquid material through the pores thereof, the wall surfaces of the said pores providing a substantially continuous surface over which the liquid material may flow, and (b) is rotatable to subject the liquid material flowing therethrough to a mean acceleration of at least 150 m $sec^{-}$ as it flows away from the said axis. Preferably the said wall surfaces provide a tortuous surface over which the liquid material may flow. Typical structures which allow passage of a liquid material through the pores thereof include *inter alia* foraminate, cribreform, perforated, orificiated or gauze-like structures.

Preferably the permeable element has a large interfacial area.

By "interfacial area" we mean the surface of the permeable element which the liquid material may contact per unit volume of permeable element. By large interfacial area we mean an interfacial area of at least 100 $m^{-1}$ (i.e. $m^2/m^3$). It is often preferred that the permeable element has an interfacial area of at least 1500 $m^{-1}$ and, where the permeable element is a catalyst, preferably at least $10^6$ $m^{-1}$.

It is often preferred that the permeable element has a voidage of at least 80% more preferably at least 90% since this tends to increase the rate at which the liquid material may be treated, although we do not exclude the possibility that a permeable element having a lower voidage may be employed, for example it may be a semipermeable membrane.

By "voidage" we mean the percentage of the total volume of permeable element which is free space.

Where the liquid material to be impelled through the pores of the permeable element in apparatus according to the invention is a slurry, suspension or dispersion of particulate material which may be inorganic or organic, for example a biological material, e.g. the liquid material is a "soup" containing cells, it is preferred that the average pore size of the permeable element is big enough to allow passage of the particles in

the liquid material through the permeable element.

The speed of rotation of the permeable element is such that the liquid material is subjected to a mean acceleration of at least 100 m $sec^{-2}$, and preferably at least 1000 m $sec^{-2}$, and particularly preferably at least 5000 m $sec^{-2}$.

Mean acceleration $a_m$ is defined by the equation:

$$a_m = \left(2\pi \frac{N}{60}\right)^2 \left(\frac{r_o^2 + r_i^2}{2}\right) 1/2$$

where N is the rotational speed of the permeable element about the said axis in revolutions per minute, $r_o$ is the distance in metres from the axis of rotation to the radially inner portion of the permeable element, and $r_i$ is the distance in metres from the axis of rotation to the radially outer portion of the permeable element.

The permeable element preferably has a plane of symmetry in which the axis of rotation lies, e.g. it may be in the form of a permeable rod which is rotated about an axis perpendicular to the axis of the rod and distant the mid-point thereof. Particularly preferably the permeable element has a plurality of planes of symmetry which intersect at a line co-incident with the axis of rotation, e.g. it may be in the form of permeable rod which is rotated about an axis perpendicular to the axis of the rod and co-incident with the mid-point thereof. More particularly preferably the permeable element has an axis of symmetry which co-incides with the axis of rotation, e.g. the permeable element may be in the form of an annulus which is rotated about its axis of symmetry. Where the permeable element is in the form of an annulus the outer diameter of the annulus is typically in the range 25 cm to 5 metres, and the inner diameter is typically in the range 5 cm to 20 cm.

The permeable element may be formed from any material which has the mechanical strength to withstand the stress generated in the material during rotation of the permeable element at the rotational speeds employed.

The permeable element may be an integral whole or a plurality of discrete compounds. Where the permeable element is an integral whole it may, for example, be formed with pores, e.g. cast as a block with pores, or knitted from a filament e.g. of glass or metal; or be arranged to form pores between the parts thereof, e.g. a coil of wire. Where the permeable element comprises a plurality of discrete compounds, the individual components may be permeable, e.g. open-ended glass tubes, in which case a proportion of the pores are through the components and a proportion of the pores are between the components; or the individual components may be non-permeable, e.g. glass or metal spheres, in which case the pores of the permeable element are between the components thereof. It is often preferred that the permeable element is an integral whole and is mechanically self-supporting since this often reduces the possibility of the voidage thereof being decreased with use.

While the permeable element may have straight pores, e.g. it may comprise aligned glass tubes or a metal block with channels drilled therein, preferably it has tortuous pores through which the fluid flows, e.g. it may be a coil of woven tape, a sintered mass, knitted or woven wire cloth, a crumpled mesh, skeleton foam, e.g. Retimet (registered trade mark), a random mat of fibres, or a mass of fibres or of particles. Where particles are employed they may all have the same size and shape, or the sizes and/or shapes may be random, or the size and/or shape may be ordered. Particles which may be employed include *inter alia*, Intalox saddles ("Intalox" is a registered trade mark), ceramic chips, wire gauze pieces, or glass beads. Preferably the particles where they are employed, have a regular shape.

The permeable element may be resistant to attack by or reaction with the liquid material or it may interact physically or chemically therewith or with a component thereof, e.g. the permeable element may comprise a chromatographic support material or a catalyst. Examples of typical chromatographic support materials are alumina and silica gel. Where the permeable element interacts with the liquid material or a component thereof, the permeable element preferably is formed from a material having a sheath/core structure in which an inert core is at least partly coated with a sheath of material which interacts with the liquid material, e.g. glass spheres having a biologically active material, e.g. enzymes, chemically bound to their surfaces.

Where the permeable element is resistant to attack by or reaction with the liquid material, the permeable element typically is formed from a glass, plastics, or a chemically resistant metal, e.g. stainless steel, nickel, titanium or tantalum. Alternatively the permeable element may be formed from a material having a sheath/core structure such as a corrosion resistant coating, e.g. of glass or plastic, on a corrodible support, e.g. corrodible metal spheres.

While the permeable element is conveniently homogeneous we do not exclude the possibility that the permeable element may be a composite. For example, an annulus of glass beads may be surrounded by an annulus of wire mesh which may be surrounded by a random mat of polytetrafluoroethylene fibres.

It will be appreciated that where the permeable element is not mechanically self-supporting, e.g. it comprises an integral whole arranged to form pores between the parts thereof, or a plurality of discrete components, or is a composite, means to retain the permeable element in a desired shape and to maintain its

permeability are often necessary. The said means is preferably in the form of a member which is rotatable about the same axis as the permeable element (hereinafter "rotatable member") and in which the permeable element is disposed. Moreover, where the permeable element is mechanically self-supporting it is often preferably disposed in a rotatable member.

Where a rotatable member is employed, the permeable element may be disposed throughout or in a proportion of the rotatable member. The size of the permeable element and its disposition in the rotatable member may be determined by the density and the interfacial area of the permeable element and by the flow characteristics of the liquid material. Where the permeable element is disposed in a proportion of the rotatable member it is often preferred that the permeable element is disposed in a radially outer proportion of the rotatable member since as the distance from the axis increases the magnitude of the centrifugal forces which operate on the liquid material to form a layer increases and hence the thickness of the layer is decreased. Where the permeable element is disposed in a rotatable member which has an axis of symmetry co-incident with the axis of rotation, the permeable element is preferably distributed symmetrically around the axis so that the rotatable member is dynamically balanced when it is rotated.

The rotatable member, where it is employed, may be constructed of any material which has (a) the mechanical strength to withstand the stress generated in the material during rotation of the rotatable member at the rotational speeds employed and (b) the corrosion resistance to tolerate the environments with which the rotatable member may be in contact during use. Typical materials from which the rotatable member may be constructed include *inter alia* stainless steel, mild steel, brass, aluminium, nickel, and Monel. Choice of a suitable material will present little problem to those skilled in the art.

The speed at which the permeable element is rotated will depend *inter alia* on its porosity, the throughput of liquid material required and the radial distance over which the liquid material flows in the permeable element. The minimum speed at which the permeable element is rotated is often determined by the flow characteristics of the liquid material. The maximum speed at which the permeable element may be rotated is governed by the mechanical strength of the permeable element, and where it is employed, by the mechanical strength of the rotatable member. Where a rotatable member is employed and where it is in the form of a hollow stainless steel disc in which the permeable element is disposed, typical rotational speeds are; for a disc of 0.5 metres diameter, 1000—3000 rpm; for a disc of 1 metre diameter, 500—2000 rpm; for a disc of 1.5 metres diameter, 400—1000 rpm. As the speed of rotation increases the thickness of the layer of liquid material on the wall surfaces of the pores of the permeable element at a fixed distance from the axis of rotation decreases.

In general, the speed of rotation will be in the range 50 rpm to 10,000 rpm, preferably in the range 100 rpm to 5,000 rpm and particularly preferably in the range 500 rpm to 2000 rpm.

While the axis of rotation may be horizontal or vertical or at any angle between, it is often convenient to have the axis vertical. Where a rotatable member in the form of a hollow disc is employed, typically rotary movement is applied to it by a shaft projecting from the plane of the disc along the axis thereof, (e.g. from the top and/or bottom if the axis is vertical). The rotatable member may be rotated by, for example, a variable speed fluid drive, a pulley which may be driven by a belt from an electric motor, or by turbo-propulsion.

The design of bearings and seals for use in apparatus according to the present invention may be those well known in the engineering art, e.g. radial and thrust bearings of conventional design.

As the interfacial area for any particular permeable element is increased, the pressure drop across the permeable element increases and the possibility of fouling and flooding of the permeable element increases. Simple experiment will readily reveal a suitable permeable element for any desired speed of rotation and liquid material combination.

Flow of the liquid material through the pores of the permeable element is substantially in planes perpendicular to the axis of rotation, i.e. radial flow, although we do not exclude the possibility that there may be a small component of the flow parallel to the axis. It will be appreciated that by employing a permeable element having a radial thickness substantially greater than its axial length, e.g. an annular permeable element, and by charging the liquid material substantially uniformly along the axial thickness of the annulus the possibility that the flow of the liquid material has an axial component is reduced. For example, where the permeable element is in the shape of an annulus it typically has a ratio of thickness:outer diameter from 1:2 to 1:15 preferably from 1:4 to 1:10 e.g. it may have a diameter of 80 cm and an axial thickness of 20 cm.

At least the major proportion of the liquid material or a derivative thereof is discharged from the permeable element preferably at or adjacent to the radially outer perimeter thereof. Thus means to at least reduce the discharge of the liquid material distant the radially outer perimeter of the permeable element are provided. For example, where the permeable element is in the shape of an annulus, it may be formed with an integral skin on its two planar surfaces, or discs may be held in contact with each of the said planar surfaces, or a rotatable element,

where one is employed, may be adapted to prevent the discharge distant the radially outer perimeter of the permeable element, e.g. the rotatable member may be a hollow disc in which the annular permeable element is disposed, the planar surfaces of the permeable element forming a seal with the planar surfaces of the hollow disc.

The permeable element may be disposed in a container adapted to be pressurised with a fluid and, where the pressure of the fluid is higher than that of the liquid material at the radially outer surface of the permeable element, the fluid may be forced into the permeable element to contact the liquid material. For example, where the permeable element is in the shape of an annulus, it may be disposed in a container such that the container and the permeable element, or, where a rotatable member is employed, the rotatable member, define an annular chamber adapted to be pressurised with a fluid to a pressure higher than that of the liquid material at the radially outer surface of the permeable element. Where the fluid has a lower density than that of the liquid material, they will flow counter-currently through the permeable element.

By "fluid" we mean a substance, or mixture of substances, which is a gas or a liquid. For example, where the fluid is a gas it may be one gas or a mixture of gases, where the fluid is a liquid it may be a neat liquid or a solution of one or more solutes in a liquid, which solute may be a gas, liquid or solid.

Where counter current flow is employed it will be appreciated that means are necessary distant the axis of rotation and preferably adjacent the radially outer perimeter of the permeable element to charge the permeable element with the said fluid. Preferably the permeable element is supported within a hollow rotatable member to form a chamber between the radially outer perimeter of the permeable element and the inner surface of the rotatable member into which chamber the liquid material flows to form a liquid seal through which the said fluid may be charged to the permeable element. Where the said fluid is a mixture of components they may be delivered to the said space through the same or separate delivery means which are conveniently radially directed channels in the base of the rotatable element.

Where the permeable element is supported in a rotatable member, means to deliver the liquid material to the permeable element typically comprises an orifice in the rotatable member through which the fluid may flow. Where the rotatable member is a hollow disc the delivery means is conveniently axially disposed, although we do not exclude the possibility that it may be located intermediate the axis of rotation and the perimeter of the rotatable member distant the axis of rotation. Where the liquid material is a mixture of components, these may be delivered to the permeable element through the same or separate delivery means, e.g. they may be delivered through concentric tubes.

Where a permeable element supported in a rotatable member is employed in apparatus according to the invention, means to discharge the liquid material or a component or derivative thereof from the rotatable member typically comprises one or more orifices in the periphery of the rotatable member distant from the axis of rotation, through which orifice the fluid may issue as a spray. Where the rotatable member is a hollow disc in which an annular permeable element is disposed the orifice is conveniently in the form of a circumferentially extending slit in the wall of the hollow disc and the slit is preferably continuous.

Conveniently the permeable element, or the rotatable member, where it is employed, is mounted in a stationary fluid-collecting means e.g. a housing, in which fluid-collecting means the liquid material or a component or derivative thereof which is discharged from the permeable element may be collected. It will be appreciated that where counter current flow is effected in apparatus according to the present invention the permeable element and the rotatable member, where it is employed, will be mounted in the fluid collecting means such that the fluids discharged from the permeable element do not mix. Alternatively the permeable element or the rotatable member where it is employed, is provided with a circumferentially extending channel into which the liquid material or a component or derivative thereof flows. One or more suitably disposed stationary collecting means, e.g. a fan-tail scoop, dip into the channel and the rotational speed of the liquid material forces the liquid material or a derivative thereof through the collecting means to a suitable location.

The residence time of the liquid material within the permeable element is a function of the radial dimensions of the permeable element, the nature and permeability of the permeable element, the rotational speed, and the flow rate of the liquid material. These parameters interact with each other and affect the residence time. For example, where the radius (of a disc shaped peremeable element) is increased and the other parameters kept constant the residence time is increased; where the flow rate is increased and the other parameters kept constant the residence time is reduced; where the rotational speed is increased and the other parameters kept constant the residence time is reduced.

It will be appreciated that for the generation of a liquid surface of large area, the liquid material and/or the fluid, where it is a liquid, preferably "wets" substantially the whole of the wall surfaces of the pores of the permeable element. Wetting of the permeable element will depend to a degree on dynamic factors but will be assisted if it is favoured by equilibrium wetting conditions. Thus a liquid having a small

interfacial tension with the permeable element will tend to displace from the surface of the pores of the permeable element a liquid having a large interfacial tension with the permeable element, which displacement process is assisted by a small interfacial tension between the two liquids. To improve the "wetting" of the permeable element the wall surfaces of the pores of the permeable element are preferably coated with a wetting agent, or a wetting agent is preferably added to the liquid material. For example, where the liquid material is water-based and the pores of the permeable element have a hydrophobic surface, e.g. the permeable element is a mat of polytetrafluoroethylene fibres, suitable surfactants, e.g. sodium dodecylsulphate or a Monflur (registered trade mark) surfactant, may be added to the water.

A plurality of permeable elements, each provided with suitable fluid-collecting means, typically a housing, although we do not exclude the possibility that a circumferential channel and associated removal means as hereinbefore described may be employed, may be joined in series. It will be appreciated that suitable pumps where appropriate, may be provided in the lines interconnecting adjacent permeable elements. Conveniently the permeable elements are mounted about a common axis.

The materials and the structure of the permeable element and, where it is employed, of the rotatable member may be chosen with regard to the nature of the treatment occurring in apparatus according to the present invention. For example, where an endothermic reaction occurs in apparatus according to the present invention the permeable element and/or the rotatable member, where it is employed, may be provided with heating means, e.g. electrical resistance wires; where an exothermic reaction occurs in apparatus according to the present invention, the permeable element and/or the rotatable member, where it is employed, may be provided with cooling means, e.g. a cooling coil.

Where it is desired to heat the liquid material in the permeable element, the permeable element or a component thereof, or, where a rotatable member is employed, the rotatable member or a component thereof may be formed from (a) an electrically conducting material and subjected to an alternating electric field to effect electromagnetic heating and/or (b) a ferromagnetic material and subjected to an alternating electric field to effect inductive heating. Alternatively, where the liquid material or the permeable element or rotatable member has a suitable dipole e.g. water or a polyvinyl chloride suspension, microwave heating may be employed using suitably disposed electrodes or a coil.

Apparatus according to the present invention may, by choice of a suitable permeable element, be used for chromatographic separation, or as a chemical or biochemical reactor.

Where apparatus according to the present invention is employed in chromatography, typical chromatographic support materials from which the permeable element may be formed include inter alia silica particles, alumina particles, particles having suitable materials chemically bound thereto, polystyrene gel beads and Sephadex (registered trade mark). A variety of chromatographic separations may be effected in apparatus according to the invention including *inter alia* adsorption chromatography, liquid-liquid chromatography, gel permeation chromatography and so-called affinity chromatography.

For use in affinity chromatography, the permeable element comprises a material which has a biological affinity for a substance which it is desired to separate from the liquid material.

By the term "biological affinity" we mean the natural affinity demonstrated by biological systems in biological interactions such as antigen-antibody, enzyme-inhibitor, and hormone-carrier interactions.

Removal of the aforesaid substance from the permeable element may be effected by treating the permeable element with a suitable displacing agent or by changing the pH of ionic strength of a liquid with which the permeable element is in contact.

By this technique it is possible to isolate proteins, polysaccharides, glycoproteins, steroids, nucleic acids, other classes of naturally occurring compound, bacteria, viruses and whole cells. Where the reactions on which the biological affinity is based are reversible it is often possible to use this technique to isolate either of the reactants.

Thus where the permeable element comprises an antigen a solution containing the antibody specific to the antigen may be passed through the permeable element and the antibody-antigen complex is formed on the permeable element. The antibody may then be removed from the antibody-antigen complex, e.g. by changing the pH of a liquid flowing through the permeable element, so that one is able to isolate and purify antibody which does not contain residual antigen. Preferably the antigen is a protein although we do not exclude the possibility that it may be a smaller molecule, e.g. a steriod or sugar.

Where the permeable element comprises a suitable antibody it is possible to recover whole cells which have a specific affinity for the antibody.

Examples of antigens against which antibodies can be obtained include *inter alia* plasma proteins, enzymes and many hormones. Examples of such hormones are steroids, insulin, gonadotropins, growth hormone, ACTH, thyrotropin and parathromone.

Antibodies can be prepared by any method known *per se*, by immunising animals, by, for instance, repeated subcutaenous injections of small amounts of the appropriate antigen,

possibly combined with a so-called adjuvant, such as Freund's mineral oil emulsion, into an animal. The antibodies produced in the animal can be recovered from the blood serum of the same. The protein fraction, which contains the antibody, can be separated by conventional methods, e.g. by precipitating the serum with suitable amount of a saturated aqueous solution of ammonium sulphate, and then subjected to affinity chromatography.

Where apparatus according to the present invention is adapted to allow charging of a fluid to the permeable element distant the axis of rotation the apparatus is useful for conducting chemical reactions particularly those requiring a large volume of gas to be in contact with a liquid material. The apparatus will transport the liquid material through the permeable element whilst allowing gas to permeate the liquid material continuously during the transport until the liquid material or a derivative thereof is discharged from the permeable element.

For example, we have found that biological materials, for example those containing cells (whether alive or dead) derived from living matter may be transported through the pores of the permeable element and a plentiful supply of air, oxygen, carbon dioxide, etc. as may be desired, may be obtained in contact with the biological matter. Choice of a suitable permeable element allows treatment of "soups" and sludges and the apparatus may be used to conduct chemical reactions with or in the biological material, for example, a reaction with a living cell and an enzyme, or a reaction involving bacteria. Another example of a reaction usefully carried out in apparatus according to the present invention is a fermentation reaction especially fermentation of biological cultures which require efficient aerations. Thus the apparatus herein described may be used as an apparatus in which a range of micro-organisms may be handled having at least many of the necessary performance characteristics described in "Laboratory Practice" October 1965 pp 1150.

Where the permeable element has catalytic properties the apparatus according to the invention may be employed as a reactor. For example, the permeable element may comprise an immobilised enzyme e.g. a proteolase, amylolase, dehydrogenase, hydroxylase, aminoacylase, or glycolase, may be chemically bound to or encapsulated in a suitable support such that typical enzymatic reactions may be effected in apparatus according to the present invention. For use in catalytic reactions, the permeable element is often particulate and the particles often have a sheath/core structure.

Where a suitable support having a biological material chemically attached thereto is employed in apparatus according to the present invention, the biological material may be attached to the suitable support by techniques known in the art. For example, a covalent bond may be formed directly between the biological material and the suitable support, or by chemical modification of the suitable support, e.g. reaction of cyanogen bromide with hydroxyl groups, e.g. of cellulose, to form iminocarbonates. Preferably however, coupling agents, e.g. glutaraldehyde, 1-fluoro-2-nitroazidobenzene, cyanuric chloride or silanes are employed to chemically attach the biological material to the suitable support.

Where the suitable support is formed from a polymeric material the polymer may be selected so that it contains, or can be provided with, suitable reactive groups such as amino groups, hydroxyl groups and carboxylic groups, to readily make possible the binding of biological material to the polymer by bridges with covalent linkages.

Where the suitable support is formed of inorganic material it often has available oxide or hydroxide groups which may be used to form bonds with the biological material; preferably, however, coupling agents are employed to effect bonding between the inorganic material and the biological material and more preferably the coupling agents are silanes.

Silane coupling agents are usually molecules which possess two different kinds of reactivity. They are organo-functional and silicon-functional silicon compounds in which the silicon portion of the molecule has an affinity for oxide and hydroxide groups of inorganic materials such as glass, silicates, and metals, while the organic portion of the molecule is tailored to combine with many organic functional groups. In theory, the variety of possible organo-functional silanes useful in this invention is limited only by the number of known organo-functional groups and the available sites on the biological material. A multitude of different silane coupling agents can be used as shown by the general formula $(Y'R')_nSiR_{4-n}$ wherein Y' is a member selected from the group consisting of amino, carbonyl, carboxy, isocyano, diazo, isothiocyano, nitroso, epoxy, halocarbonyl; R is a member selected from the group consisting of lower alkoxy, phenoxy, and halo: R' is a member selected from the group consisting of lower alkyl, lower alkylphenyl, and phenyl; and n is an integer having a value of 1—3. Useful silane coupling agents may be represented by the formula: $Y_nSiR_{4-n}$ wherein Y is a member selected from the group consisting of amino, carbonyl, carboxy, hydroxyphenyl, and sulfhydryl; R is a member selected from the group consisting of lower alkoxy, phenoxy, and halo; and n is an integer having a value of 1—3. However, commercially available coupling agents often have the formula

$$XCH_2CH_2CH_2Si(OR)_3$$

wherein R is ethyl or methyl and X is a reactive organic group, tailored to match the reactivity of the system in which it is to be used. X is typi-

cally $NH_2$—, $NH_2CH_2CH_2NH$—, HS—,

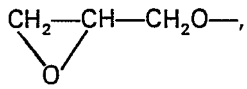

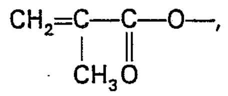

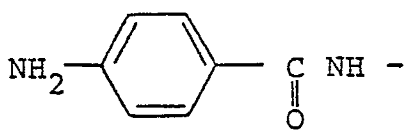

Epoxy silanes such as ω-glycidoxypropyl-trimethoxy silane and amino silanes such as N-β - aminoethyl - γ - aminopropyl - trimethoxy-silane, N - β - aminoethyl - (α - methyl - γ-aminopropyl)dimethoxy - methylsilane, γ - amino-propyl - triethoxysilane, γ(p-aminobenzamido)-propyl-triethoxysilane, p - aminobenzene - trimethoxysilane are preferred. These coupling agents may be modified before reaction with the agent, e.g. alkyl amino groups may be reacted with carbodiimides or may be converted to isothio-cyanoalkylsilane groups by reaction with thio-phosgene, and aromatic groups may be converted to diazonium groups by reaction with nitrous acid.

Where silane coupling agents are employed they often form covalent bonds with oxide or hydroxide groups on the inorganic material.

The coupling agent is conveniently applied to the suitable support from a solvent solution. Preferably an aromatic or aliphatic solvent is employed, particularly good solvents are toluene, benzene, xylene, and high boiling hydrocarbons. While the silane coupling agents are soluble in polar solvents such as alcohol and water, polar solvents should be avoided because they tend to retard bonding of the silane to the permeable element. Also, aldehydes, ketones, acids, esters, or alkyl chlorides should be avoided as solvents because they tend to react with the silanes.

Bonding of the biological material to the suitable support is conveniently a two step process:— the first step involving bonding of the coupling agent to the suitable support and the second step involving the bonding of the biological material to the coupling agent. The quantity of biological material which may be coupled depends on the amount and nature of the functional groups on the coupling agent and the biological material. The coupling agent is preferably chosen so that the biological activity of the biological material is not modified or destroyed.

Where the biological material is a protein coupling of the protein to the suitable support is accomplished by reaction of the coupling agent with the terminal groups and/or pendant functional groups of the protein. Often the proteins to be coupled have sufficient numbers of functional side chains to allow one to select the type of reaction through which coupling can be most easily accomplished. The most frequent site of coupling is through the N-terminal amino group and pendant amino groups contained in lysine residues. The imidazoyl groups of histidine, and the guanidinyl groups in arginine are other amine functions which may be involved in coupling. Covalent coupling may also be effected through the carboxylic acid residues present in glutamic and aspartic acid and through the C-terminal ends of the protein molecule. Another site for coupling is the hydroxyphenyl groups present in tyrosyl residues which reacts readily with a diazonium reagent. Alternatively, coupling can also be effected through formation of a disulphide linkage with the sulphydryl groups present in cysteine residues. Cysteine sulphydryls are also sufficiently nucleophilic to react with glutaraldehyde and other cross-linking reagents.

To reduce the possibility of modifying or destroying the biological activity of a protein it is important that the biologically active sites of the protein are not blocked by the coupling reaction. One of the ways to avoid blocking the active site is to use a coupling technique which does not involve reaction of the functional group present at the biologically active site. The active site can be protected during coupling reactions by employing a covalent blocking reagent, or by performing the reaction in the presence of a substrate or specific inhibitors. Such protection techniques are known. Furthermore the coupling agent should be such that conditions employed for coupling do not destroy the biological activity of the protein, e.g. where pepsin is being attached it must be acid pH since pepsin denatures rapidly above pH 5. However we do not exclude the possibility that the biological activity may be modified by the coupling action, e.g. the pH maxima, Michaelis constant and substrate specificity may be altered thus affording potential for manipulation of enzymes for specific catalytic purposes. A plurality of biologically active materials may be bonded concurrently or sequentially to the suitable support.

The invention will be further described by reference to the accompanying drawings which show, by way of example only, one embodiment of apparatus according to the present invention.

In the drawings, Figure 1 is a longitudinal cross-section of a reactor;

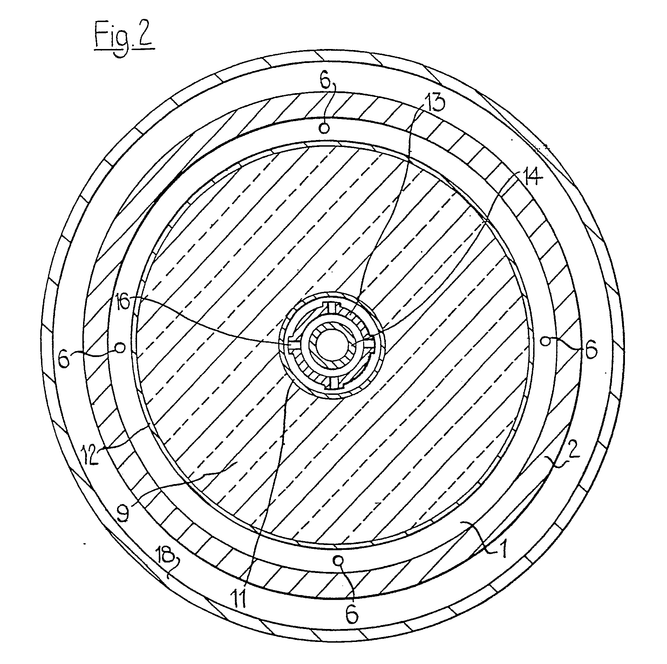

Figure 2 is a transverse cross-section on a different scale on the line AA of Figure 1.

In Figures 1 and 2 a hollow disc is formed with a stainless steel base 1 and wall 2 and a Perspex lid 3 bolted to the wall 2 ("Perspex" is a registered trade mark). Wire meshes 11 and 12 are mounted in the hollow disc and with portions of the base 1 and lid 3 form a rotatable member in which a permeable element 9 formed from a Cambrelle mat is supported

("Cambrelle" is a registered trade mark). The base 1 is provided with a hollow shaft 4 communicating with four radial channels 5 which lead via ports 6 to an annular chamber defined by the wall 2, the wire mesh 12 and portions of the base 1 and lid 3. The concentric tubes 13 and 14 project through the lid 3 via a gas-tight seal 15. The outer tube 13 communicates with four fan sprays 16 through which the liquid material may be fed to the mat 9. The hollow shaft 4 is rotatably mounted on roller bearings in a bearing housing 17 which is attached to a stationary housing of stainless steel 18 provided with a port 19. An electric motor (not shown) provides the drive to the hollow shaft in a "vee" belt drive.

In operation, the hollow disc is rotated, a liquid is fed via the tube 13 to the mat 9, moves radially outward to fill the chamber between the wire mesh 12 and the wall 2 and is expelled through the passage defined by the lip 7 and the groove 8. A gas at high pressure is fed into the apparatus through the hollow shaft 4 and the channels 6 and enters the annular chamber between the wall 2 and the wire mesh 12. The liquid in the chamber between the wall 2 and the outer mesh 12 prevents escape of gas at the wall 2 and the gas is forced through the pores of the permeable element counter-current to the liquid to escape via the tube 14. The liquid collects in the housing 18 and may be run off through pore 19 as desired.

Example 1

1000 litres of beads of DEAE-Sephadex A—25, buffered with 0.1M phosphate buffer (pH 7.0), and 1500 litres of an aqueous solution of native aminoacylase ($334 \times 10^6$ units) were stirred at 35°C for 10 hours. The DEAE-Sephadex-aminoacylase complex was filtered off washed with water and 0.2M acetyl-DL-methionine solution. The activity of the complex was 180,000 units per litre of preparation.

A hollow disc as illustrated in Figures 1 and 2 was packed with the complex which formed an annular permeable element of internal radius 4 cm and external radius 9 cm. The disc was rotated at 500 rpm and a 0.2M solution of acetyl-DL-methionine at 50°C was impelled through the permeable element at a flow rate of 20 ml/min. From the liquid discharged from the disc it was found by polarimetry that approximately 65% of the acetyl-L-methionine had been hydrolysed to L-methionine.

## Claims

1. Apparatus for treating a liquid material, which comprises a permeable element (9) of annular section which is rotatable about the axis of the annulus, means (16) to charge the liquid material to the inner surface of the permeable element and means (18) to collect the liquid material or a component or derivative thereof discharged from the outer surface of the permeable element by rotation of the element, characterised in that the permeable element (a) comprises a catalyst for carrying out a chemical reaction in or on the liquid material or is formed from a chromatographic support material, and (b) allows the liquid material to flow through its pores.

2. Apparatus according to claim 1 characterised in that the permeable element (9) is disposed within a hollow rotatable member (1, 2, 3).

3. Apparatus according to claim 2 characterised in that the permeable element (9) is supported in the rotatable member (1, 2, 3) to form a chamber between the radially outer perimeter of the permeable element and the inner surface of the rotatable member into which chamber the liquid material flows to form a liquid seal through which a fluid having a lower density than that of the liquid material, may be charged to the permeable element so that they flow counter-currently therethrough.

4. Apparatus according to any one of the preceding claims characterised in that the permeable element is an immobilised enzyme.

5. A process for treating a liquid material which process comprises (i) charging the liquid material to a permeable element which allows passage of the liquid material through the pores thereof, the wall surfaces of the pores providing a substantially continuous path over which the liquid material may flow, (ii) rotating the permeable element about an axis to subject the liquid material to a mean acceleration of at least 150 m $sec^{-2}$ as it flows through the said pores away from the said axis and (iii) collecting the liquid material or a component or derivative thereof from the permeable element, characterised in that the permeable element (a) comprises a catalyst for carrying out a chemical reaction in or on the liquid material or is formed from a chromatographic support material, and (b) allows the liquid material to flow through its pores.

6. A process according to claim 5 characterised in that the liquid material is a dispersion, suspension or slurry of one or more solids in a liquid.

7. A process according to claim 6 characterised in that the solid comprises biological material.

## Revendications

1. Appareil de traitement d'une matière liquide, qui comprend un élément perméable (9) à section annulaire qui est rotatif autour de l'axe de l'anneau, un dispositif (16) pour amener la matière liquide à la surface intérieure de l'élément perméable et un dispositif (18) pour collecter la matière liquide ou un composant ou dérivé de celle-ci évacué à la surface extérieure de l'élément perméable par la rotation de l'élément, caractérisé en ce que l'élément perméable (a) comprend un catalyseur pour

exécuter une réaction chimique dans ou sur la matière liquide ou est formé d'une matière de support chromatographique et (b) laisse la matière liquide s'écouler à travers ses pores.

2. Appareil suivant la revendication 1, caractérisé en ce que l'élément perméable (9) est disposé à l'intérieur d'un organe rotatif creux (1, 2, 3).

3. Appareil suivant la revendication 2, caractérisé en ce que l'élément perméable (9) est supporté dans l'organe rotatif (1, 2, 3) pour former une chambre entre le périmètre radialement extérieur de l'élément perméable et la surface intérieure de l'organe rotatif, chambre dans laquelle la matière liquide s'écoule pour former un joint liquide à travers lequel un fluide ayant une densité inférieure à celle de la matière liquide peut être amené à l'élément perméable de manière qu'ils s'écoulent à contre-courant à travers l'élément.

4. Appareil suivant l'une quelconque des revendications précédentes, caractérisé en ce que l'élément perméable est une enzyme immobilisée.

5. Procédé de traitement d'une matière liquide, lequel procédé comprend (i) l'amenée de la matière liquide à un élément perméable qui permet le passage de la matière liquide à travers ses pores, les surfaces des parois des pores constituant un trajet sensiblement continu sur lequel la matière liquide peut s'écouler, (ii) la mise en rotation de l'élément perméable autour d'un axe pour soumettre la matière liquide à une accélération moyenne d'au moins 150 m $s^{-2}$ tandis qu'elle s'écoule à travers les pores à partir de cet axe et (iii) la collecte de la matière liquide ou d'un constituant ou dérivé de celle-ci à partir de l'élément perméable, caractérisé en ce que l'élément perméable (a) comprend un catalyseur pour exécuter une réaction chimique dans ou sur la matière liquide ou est formé d'une matière de support chromatographique et (b) laisse la matière liquide s'écouler à travers ses pores.

6. Procédé suivant la revendication 5, caractérisé en ce que la matière liquide est une dispersion, une suspension ou bouillie d'un ou plusieurs solides dans un liquide.

7. Procédé suivant la revendication 6, caractérisé en ce que le solide comprend une matière biologique.

**Patentansprüche**

1. Vorrichtung zur Behandlung eines flüssigen Materials, die

ein durchlässiges Bauteil (9) mit kreisringförmigem Querschnitt, das um die Achse des Kreisringes herum drehbar ist,

eine Einrichtung (16) für die Zuführung des flüssigen Materials zu der Innenfläche des durchlässigen Bauteils und

eine Einrichtung (18) zum Sammeln des flüssigen Materials oder eines Bestandteils oder Derivats davon, das durch Drehung des durchlässigen Bauteils von dessen Außenfläche abgelassen wird,

enthält, dadurch gekennzeichnet, daß das durchlässige Bauteil

(a) einen Katalysator für die Durchführung einer chemischen Reaktion in oder an dem flüssigen Material enthält oder aus einem chromatographischen Trägermaterial gebildet ist und

(b) einen Durchfluß des flüssigen Materials durch seine Poren zuläßt.

2. Vorrichtung nach Anspruch 1, dadurch gekennzeichnet, daß das durchlässige Bauteil (9) innerhalb eines hohlen, drehbaren Baugliedes (1, 2, 3) angeordnet ist.

3. Vorrichtung nach Anspruch 2, dadurch gekennzeichnet, daß das durchlässige Bauteil (9) in dem drehbaren Bauglied (1, 2, 3) unter Bildung eines Kammer zwischen der radial äußeren Begrenzung des durchlässigen Bauteils und der Innenfläche des drehbaren Baugliedes gehalten bzw. getragen wird, wobei das flüssige Material in diese Kammer unter Bildung einer Sperrflüssigkeit hineinfließt, durch die hindurch dem durchlässigen Bauteil ein Fluid, dessen Dichte geringer ist als die Dichte des flüssigen Materials, zugeführt werden kann, so daß sie im Gegenstrom durch das durchlässige Bauteil hindurchfließen.

4. Vorrichtung nach einem der vorhergehenden Ansprüche, dadurch gekennzeichnet, daß das durchlässige Bauteil ein immobilisiertes Enzym ist.

5. Verfahren zur Behandlung eines flüssigen Materials, bei dem

(i) das flüssige Material einem durchlässigen Bauteil zugeführt wird, das einen Durchfluß des flüssigen Materials durch seine Poren zuläßt, wobei die Wandoberflächen der Poren einen im wesentlichen kontinuierlichen Weg, über den das flüssige Material fließen kann, zur Verfügung stellen,

(ii) das durchlässige Bauteil um eine Achse herum gedreht wird, um das flüssige Material einer mittleren Beschleunigung von mindestens 150 m · $s^{-2}$ zu unterziehen, während es durch die Poren hindurch von der Achse wegfließt, und

(iii) das flüssige Material oder ein Bestandteil oder Derivat davon aus dem durchlässigen Bauteil aufgefangen bzw. gesammelt wird,

dadurch gekennzeichnet, daß das durchlässige Bauteil

(a) einen Katalysator für die Durchführung einer chemischen Reaktion in oder an dem flüssigen Material enthält oder aus einem chromatographischen Trägermaterial gebildet ist und

(b) einen Durchfluß des flüssigen Materials durch seine Poren zuläßt.

6. Verfahren nach Anspruch 5, dadurch gekennzeichnet, daß das flüssige Material eine

Dispersion, Suspension oder Aufschlämmung eines oder mehr als eines Feststoffs in einer Flüssigkeit ist.

7. Verfahren nach Anspruch 6, dadurch gekennzeichnet, daß der Feststoff aus biologischem Material besteht.

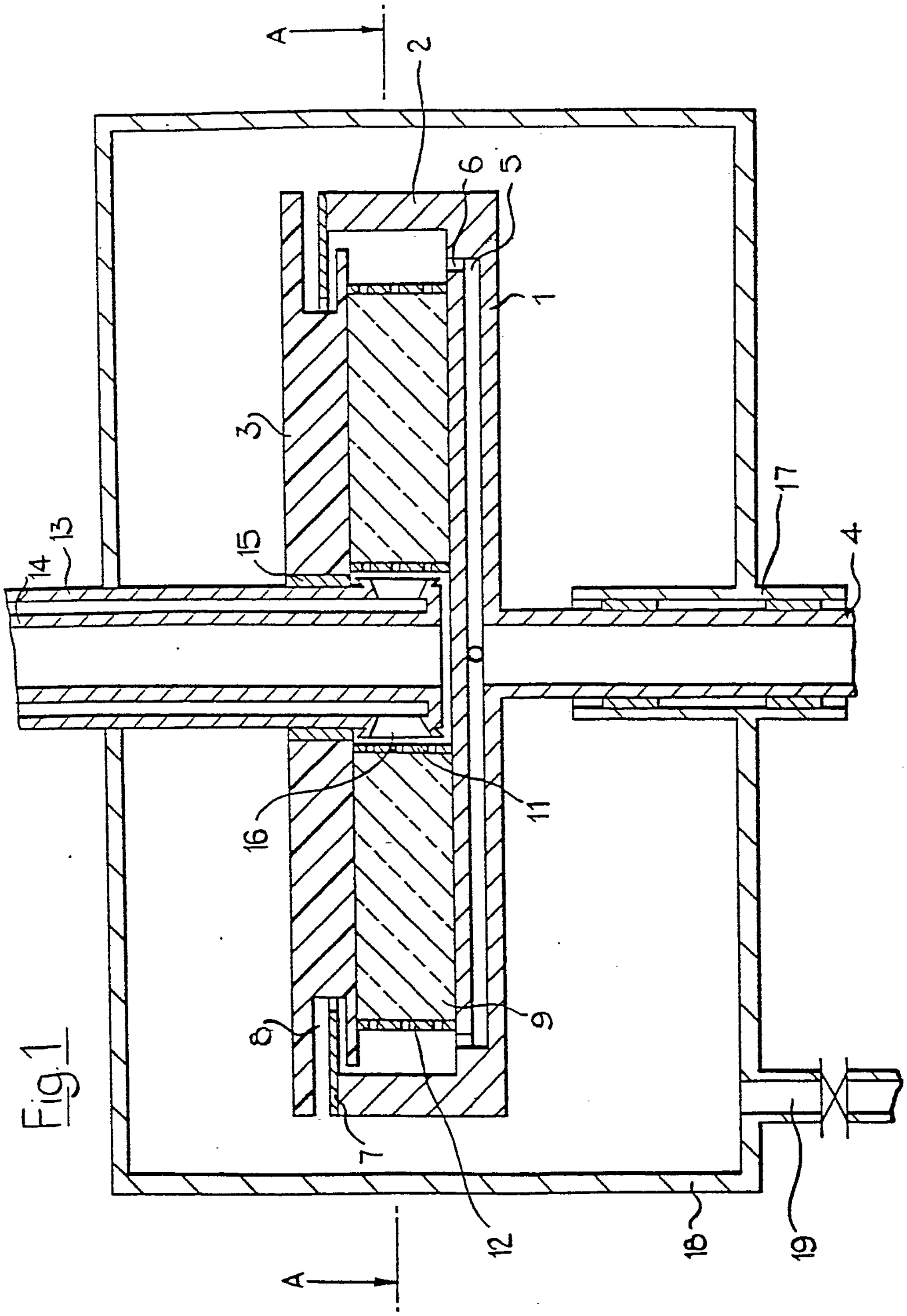


Fig.1

Fig. 2